(19) Europäisches Patentamt
European Patent Office
Office européen des brevets

(11) Veröffentlichungsnummer: **0 199 672**
**B1**

(12) # EUROPÄISCHE PATENTSCHRIFT

(45) Veröffentlichungstag der Patentschrift: **01.06.88**

(21) Anmeldenummer: **86810164.3**

(22) Anmeldetag: **07.04.86**

(51) Int. Cl.⁴: **C 07 C 143/68**, C 07 C 161/00, C 07 D 333/22, C 08 F 220/38, G 03 C 1/70, G 03 F 7/10, G 03 F 7/26

(54) **Oximsulfonate mit reaktiven Gruppen.**

(30) Priorität: **12.04.85 CH 1580/85**

(43) Veröffentlichungstag der Anmeldung: **29.10.86 Patentblatt 86/44**

(45) Bekanntmachung des Hinweises auf die Patenterteilung: **01.06.88 Patentblatt 88/22**

(84) Benannte Vertragsstaaten: **BE CH DE FR GB IT LI NL SE**

(56) Entgegenhaltungen:
**EP - A - 0 044 115**
**EP - A - 0 139 609**
**DE - A - 2 257 062**
**FR - A - 2 236 843**
**US - A - 4 289 865**
**US - A - 4 353 787**
**US - A - 4 504 372**

(73) Patentinhaber: **CIBA-GEIGY AG, Klybeckstrasse 141, CH-4002 Basel (CH)**

(72) Erfinder: **Dietliker, Kurt, Dr., Ave Jean-Marie Musy 6, CH-1700 Fribourg (CH)**
Erfinder: **Rutsch, Werner, Dr., Avenue Weck-Reynold 1, CH-1700 Fribourg (CH)**
Erfinder: **Berner, Godwin, Dr., Sommerhalde 5, CH-4102 Binningen (CH)**
Erfinder: **Hunziker, Max, Dr., Bösingenfeldstrasse 34, CH-3178 Bösingen (CH)**
Erfinder: **Demmer, Christopher George, Dr., 20 Wolsey Way, Cambridge CB1 3JG (GB)**

**EP 0 199 672 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Sulfonsäureester von Oximen. Diese enthalten im Sulfonsäurerest eine polymerisationsfähige Gruppe oder eine Gruppe, die mit funktionellen Gruppen eines Polymers oder dessen Vorstufen reagieren kann. Die Erfindung betrifft auch die so erhältlichen Polymeren und deren Verwendung zur photochemischen Bilderzeugung.

Aus der EP-A1-44115 und aus der EP-A1-139 609 sind Oximsulfonate bekannt, die thermisch oder photolytisch unter Bildung der freien Sulfonsäuren gespalten werden und die als Härtungskatalysatoren für säurehärtbare Lacke verwendet werden können. Hierfür verwendet man vorzugsweise Ester von nicht-reaktiven Sulfonsäuren, um Reaktionen mit Lackharzen zu vermeiden, welche die Härtung der Lacke stören können.

Wenn man jedoch die Oxime mit solchen Sulfonsäuren verestert, die eine funktionelle Gruppe enthalten, die entweder eine polymerisierbare oder polykondensierbare Gruppe ist, oder mit bestimmten Polymeren unter Verknüpfung reagieren kann, so kann man daraus Polymere herstellen, die eine Oximsulfonat-Seitengruppe besitzen und die durch Bestrahlung oder durch Erhitzen in Polymere mit Sulfonsäure-Seitengruppen übergeführt werden können. Für solche polymere Oximsulfonate ergeben sich eine Reihe von interessanten Verwendungsmöglichkeiten.

Die vorliegende Erfindung befasst sich zunächst mit den Monomeren, also mit Oximsulfonaten, die im Sulfonsäurerest bestimmte funktionelle Gruppen tragen. Es handelt sich dabei um Verbindungen der Formel I,

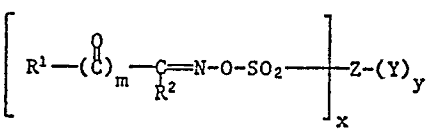 (I)

worin m=0 oder 1, x=1 oder 2 und y=1 oder 2 ist,

$R^1$ $C_1$-$C_{12}$-Alkyl, $C_1$-$C_4$-Halogenalkyl, $C_5$-$C_{12}$-Cycloalkyl, unsubstituiertes oder durch einen oder mehrere der Substituenten Halogen, $C_1$-$C_{16}$-Alkyl, $C_1$-$C_4$-Alkoxy, Phenoxy, Phenyl oder Nitro substituiertes $C_6$-$C_{10}$-Aryl, Furyl, Thienyl, $C_7$-$C_{12}$-Aralkyl, $C_1$-$C_8$-Alkoxy, $C_5$-$C_8$-Cycloalkoxy, Phenoxy oder CN bedeutet,

$R^2$ eine der für $R^1$ gegebenen Bedeutungen hat oder $C_2$-$C_6$-Alkanoyl, Benzoyl, $C_2$-$C_5$-Alkoxycarbonyl, Phenoxycarbonyl, $-N(R^3)(R^4)$, Morpholino oder Piperidino bedeutet oder $R^1$ und $R^2$ zusammen mit den Atomen, an die sie gebunden sind, einen 5-8-gliedrigen Ring bilden, der durch 1 oder 2 Benzoreste anneliert sein kann, wobei $R^3$ Wasserstoff, $C_1$-$C_{12}$-Alkyl, Phenyl, $C_2$-$C_6$-Alkanoly oder Benzoyl und $R^4$ Wasserstoff, $C_1$-$C_{12}$-Alkyl oder Cyclohexyl bedeuten,

Y eine polymerisierbare ethylenisch ungesättigte Gruppe oder Epoxidgruppe oder eine Gruppe $-OH$, $-NHR^5$, $-COOH$, $-COOR^6$, $-COCl$, $-CH_2Hal$, $-SO_2Cl$ oder $-NCO$ ist, worin $R^5$ Wasserstoff, $C_1$-$C_6$-Alkyl oder Phenyl bedeutet, $R^6$ $C_1$-$C_6$-Alkyl oder Phenyl und Hal Chlor oder Brom bedeutet, und

Z ein (x+y)-wertiges organisches Zwischenglied ist, das den Sulforest $-SO_2-$ mit der Gruppe Y verbindet.

Das Bindeglied Z ist dabei vorzugsweise eine (x+y)-wertige aromatische, aliphatische, cycloaliphatische oder araliphatische Gruppe, die durch O, S, CO, $SO_2$ oder $NR^5$ unterbrochen oder durch Halogen, Alkyl, Phenyl, Hydroxy, Alkoxy oder Aryloxy substituiert sein kann.

$R^1$, $R^2$, $R^3$, $R^4$, $R^5$ und $R^6$ als Alkyl können gerade- oder verzweigtkettiges Alkyl sein und können im Rahmen der gegebenen C-Anzahl z. B. Methyl, Ethyl, Propyl, Isopropyl, n-Butyl, s-Butyl, t-Butyl, n-Pentyl, Isoamyl, n-Hexyl, 2-Ethylbutyl, n-Octyl, 2-Ethylhexyl, n-Decyl oder n-Dodecyl sein.

$R^1$ oder $R^2$ als $C_1$-$C_4$-Halogenalkyl kann z. B. Chlormethyl, Trichlormethyl, Trifluormethyl oder 2-Brompropyl sein.

$R^1$ und $R^2$ als Cycloalkyl können z. B. Cyclopentyl, Cyclohexyl, Cyclooctyl oder Cyclododecyl sein.

$R^1$ und $R^2$ als unsubstituiertes oder substituiertes Aryl können z. B. Phenyl, 4-Fluorphenyl, 4-Chlorphenyl, 2,4-Dichlorphenyl, 2,6-Dichlorphenyl, p-Tolyl, 2,4-Dimethylphenyl, 4-Isopropylphenyl, 4-t-Butylphenyl, 4-Dodecylphenyl, 4-Methoxyphenyl, 4-Butoxyphenyl, 4-Phenoxyphenyl, 4-Biphenylyl, 3-Nitrophenyl, 1- oder 2-Naphthyl, 5-Chlor-1-naphthyl, 6-Brom-2-naphthyl, 4-Nitro-1-naphthyl oder 6-Methoxy-2-naphthyl sein. $R^1$ als Furyl oder Thienyl ist vorzugsweise 2-Furyl oder 2-Thienyl.

$R^1$ und $R^2$ als Aralkyl können z. B. Benzyl, 1- oder 2-Phenylethyl, 3-Phenylpropyl, 2-Phenylisopropyl, 2-Phenylhexyl oder Naphthylmethyl sein.

$R^1$ oder $R^2$ als Alkoxy ist vorzugsweise Methoxy oder Ethoxy. $R^1$ oder $R^2$ als Cycloalkoxy ist vorzugsweise Cyclohexyloxy. $R^2$ als Alkoxycarbonyl ist vorzugsweise Methoxycarbonyl oder Ethoxycarbonyl. Wenn $R^1$ und $R^2$ zusammen mit den Atomen, an die sie gebunden sind, einen 5-8-gliedrigen Ring bilden, so kann das ein isocyclischer oder heterocyclischer Ring sein, wie z. B. ein Cyclopentan-, Cyclohexan-, Cycloheptan-, Pyran- oder Piperidinring. Dieser Ring kann durch Benzoreste anneliert sein. Beispiele hierfür sind ein Tetrahydronaphthalin-, Dihydroanthracen-, Indan-, Chroman-, Fluoren-, Xanthen- oder Thioxanthenringsystem. Der Ring kann auch Carbonylgruppen enthalten. Beispiele hierfür sind Benzochinon-, Naphthochinon- oder Anthrachinonreste.

$R^2$ und $R^3$ als Alkanoyl können z. B. Acetyl, Propionyl, Butyryl oder Hexanoyl sein.

Y als polymerisierbare Gruppe kann eine ethylenisch ungesättigte Gruppe oder eine Epoxidgruppe sein. Die Gruppe Y kann im Molekül einmal oder zweimal vorhanden sein. Falls Y eine ethylenisch ungesättigte Gruppe ist, ist y vorwiegend 1. Beispiele für ethylenisch ungesättigte polymerisierbare Gruppen sind Vinyl, Allyl, Vinyloxy, Vinyloxycarbonyl, Allyloxy, (Di)allylamino, (Meth)-acryloxy, (Meth)acrylamido, Maleinimido oder Itaconimido. Beispiele für polymerisierbare Epoxidgruppen sind die Gruppen

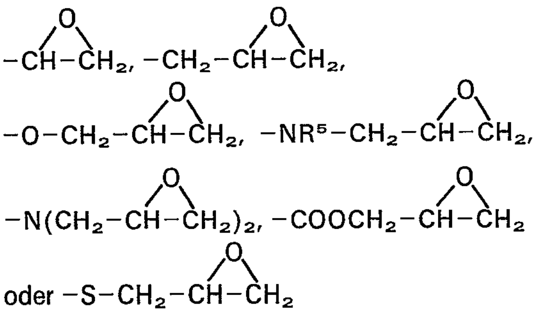

oder

Bevorzugt ist y 1 und Y eine Gruppe der Formel
$-(CH_2)_n-C(R^7)=CH_2$,
$-O-(CH_2)_n-C(R^7)=CH_2$,
$-O-CO-C(R^7)=CH_2$,
$-NH-CO-C(R^7)=CH_2$,
$-N(CH_2CH=CH_2)_2$,

$-CO-O-(CH_2)_n-C(R^7)=CH_2$,

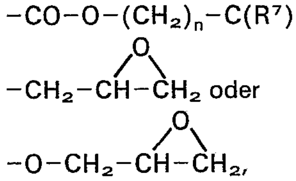

oder

wobei $R^7$ H oder $CH_3$ und n 0 oder 1 bedeuten.

Verbindungen der Formel I, in denen Y eine solche ethylenisch ungesättigte Gruppe ist, können radikalisch homo- oder copolymerisiert werden.

Wenn Y eine Epoxidgruppe oder eine Gruppe $-OH$, $-NHR^5$, $-COOH$, $-COOR^6$, $-COCl$, $-CH_2Hal$, $-SO_2Cl$ oder $-NCO$ ist, so kann dieser Rest einmal oder zweimal im Molekül vorhanden sein, je nach der Bedeutung von y. Wenn y=2 ist, so eignen sich diese Verbindungen für eine Polykondensation oder Polyaddition mit einer anderen difunktionellen Verbindung. Ist z. B. y=2 und Y=$-OH$, so sind die Verbindungen der Formel I Diole, die sich mit Dicarbonsäuren oder deren Derivaten zu Polyestern oder mit Diepoxiden zu Polyethern oder mit Diisocyanaten zu Polyurethanen umsetzen lassen. Sind die Verbindungen der Formel I Diamine, so lassen sie sich mit Dicarbonsäuren oder deren Derivaten zu Polyamiden, mit Diepoxiden zu Polyaminen, mit Diisocyanaten zu Polyharnstoffen umsetzen. Sind die Verbindungen der Formel I Dicarbonsäuren, so lassen sie sich mit Diaminen zu Polyamiden und mit Diepoxiden zu Polyestern umsetzen. Sind die Verbindungen Dicarbonsäureester (Y=$-COOR^6$) oder Dicarbonsäurechloride (Y=$-COCl$), so lassen sie sich mit Diolen zu Polyestern und mit Diaminen zu Polyamiden umsetzen. Sind die Verbindungen Dihalogenide, so lassen sie sich mit Diolen zu Polyethern und mit Diaminen zu Polyaminen umsetzen. Sind die Verbindungen Diisocyanate (Y=$-NCO$), so lassen sie sich mit Diolen zu Polyurethanen und mit Diaminen zu Polyharnstoffen umsetzen. Dies ist nur eine Auswahl von Möglichkeiten zur Bildung von linearen Polymeren aus difunktionalen Verbindungen der Formel I. Der Fachmann hat es in der Hand, für den benötigten Zweck eine geeignete Poly-Reaktion und geeignete Reaktionspartner auszusuchen.

Wenn y=1 ist und Y eine Epoxidgruppe oder eine Gruppe $-OH$, $-NHR^5$, $-COOH$, $-COOR^6$, $-COCl$, $-CH_2Hal$, $-SO_2Cl$ oder $-NCO$ ist, so eignen sich die Verbindungen der Formel I zu einer Umsetzung mit bestimmten Polymeren unter Verknüpfung des Moleküls der Formel I mit dem Polymeren.

Wenn z. B. das Polymere freie Hydroxylgruppen hat, wie dies beispielsweise bei Cellulose und Cellulosederivaten, bei Teilestern des Polyvinylalkohols, bei Copolymerisaten des 2-Hydroxyethylacrylates, bei Novolak-Harzen oder bei Epoxidharzen der Fall ist, so lassen sich z.B. Verbindungen der Formel I in denen Y $-COCl$, $-CH_2Hal$, $-NCO$, $-SO_2Cl$ oder $-COOR^6$ ist, damit umsetzen.

Wenn das Polymere Anhydridgruppen enthält, wie z. B. Copolymerisate des Maleinsäureanhydrides, so kann man eine Verknüpfung mit Verbindungen der Formel I erreichen, worin Y $-OH$ oder $-NHR^5$ ist. Wenn das Polymere freie Carboxylgruppen enthält, wie z. B. Copolymere der Acrylsäure, so lassen sich damit z. B. Verbindungen der Formel I umsetzen, in denen Y eine Epoxidgruppe ist. Wenn das Polymere Epoxidgruppen enthält, wie z. B. Copolymerisate des Glycidylmethacrylates, so kann man dieses z. B. mit Verbindungen der Formel I umsetzen, worin Y $-NHR^5$ oder $-COOH$ ist.

Beispiele für Zwischenglieder Z sind 2-, 3- oder 4-wertige aromatische oder aromatisch-aliphatische Gruppen, wie z. B. die Gruppen der Formeln

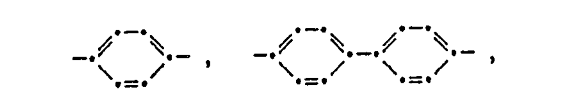

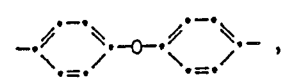

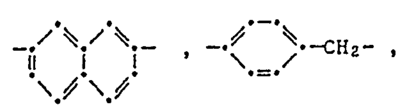

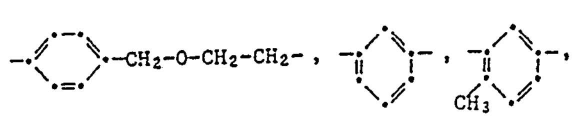

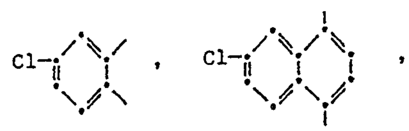

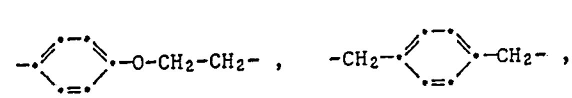

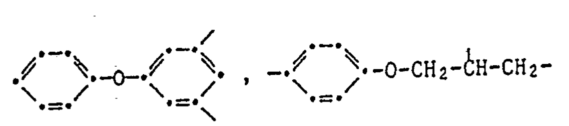

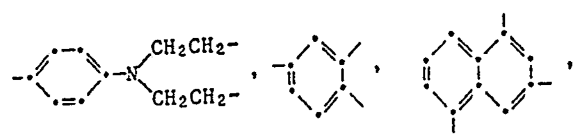

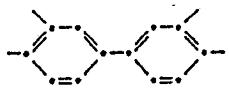 , 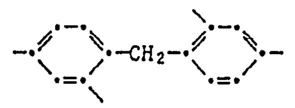 .

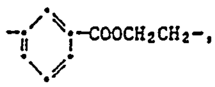 , 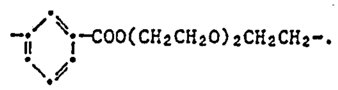 .

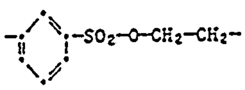 , 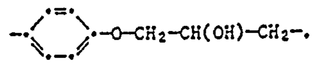 .

Beispiele für aliphatische oder cycloaliphatische Reste Z sind die Gruppen $-CH_2-$, $-(CH_2)_2-$, $-(CH_2)_3-$, $-(CH_2)_4-$, $-(CH_2)_6-$, $-(CH_2)_8-$, $-CH_2CH_2-O-CH_2CH_2-$, $-(CH_2CH_2O)_2CH_2CH_2-$, $-CH_2CH_2SCH_2CH_2-$, $-CH_2CH_2NHCH_2CH_2-$, $-CH_2-CH(CH_3)-CH_2-$,

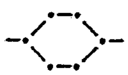 , 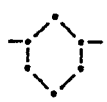 , 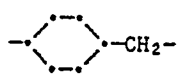 ,

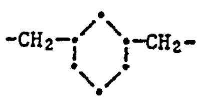 , 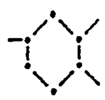 , 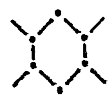 ,

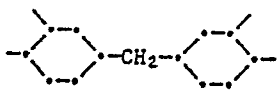 ,

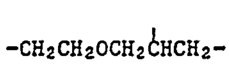 , 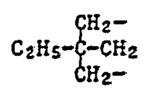 ,

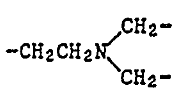 ,

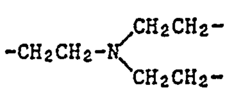 , 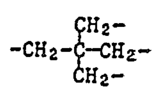 .

Wenn x und y 1 sind, ist Z ein zweiwertiger Rest und ist vorzugsweise durch $C_1$-$C_{16}$-Alkyl oder Halogen substituiertes $C_6$-$C_{12}$-Arylen, $C_7$-$C_{12}$-Arylenalkylen, $C_8$-$C_{12}$-Arylendialkylen, Oxydiphenylen oder gerade- oder verzweigtkettiges $C_1$-$C_{12}$-Alkylen, das durch O, S, CO, $SO_2$ oder $NR^5$ unterbrochen oder durch Phenyl, Halogen, Phenoxy, $C_1$-$C_4$-Alkoxy oder OH substituiert sein kann.

Bevorzugt sind Verbindungen der Formel I, worin $R^1$ $C_1$-$C_6$-Alkyl, $C_1$-$C_4$-Halogenalkyl, unsubstituiertes oder durch 1 oder 2 der Substituenten Cl, $C_1$-$C_4$-Alkyl, $C_1$-$C_4$-Alkoxy oder $NO_2$ substituiertes Phenyl, 2-Furyl, 2-Thienyl, $C_1$-$C_4$-Alkoxy oder $-CN$ bedeutet, $R^2$ eine der für $R^1$ gegebenen Bedeutungen hat oder Dialkylamino oder Morpholino ist, oder $R^1$ und $R^2$ zusammen mit den Atomen, an die sie gebunden sind, einen 5- oder 6-gliedrigen Ring bilden, der durch 1 oder 2 Benzoreste anneliert sein kann.

Besonders bevorzugt sind Verbindungen der Formel I, worin m=0 oder 1 ist, $R^1$ $C_1$-$C_4$-Alkyl, Trifluormethyl, Phenyl, Mono- oder Dichlorphenyl oder Methoxyphenyl ist, $R^2$ eine der für $R^1$ angegebenen Bedeutungen hat oder $-CN$ bedeutet oder $R^1$ und $R^2$ zusammen mit dem C-Atom, an das sie gebunden sind, einen Inden-, Fluoren-, Tetralin- oder Dihydroanthracenring bilden.

Beispiele für einzelne Verbindungen der Formel I sind die folgenden Verbindungen:

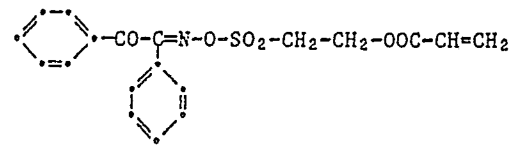

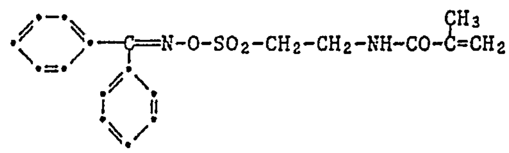

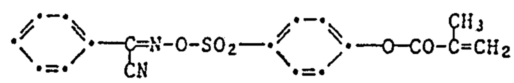

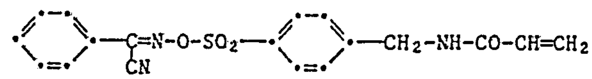

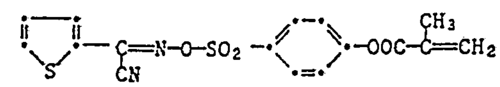

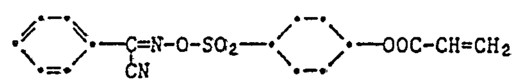

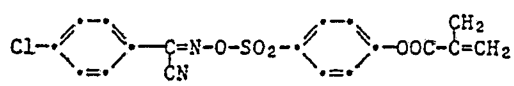

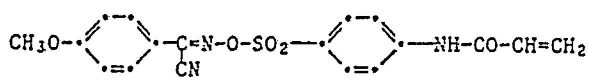

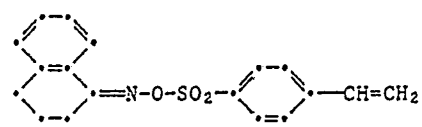

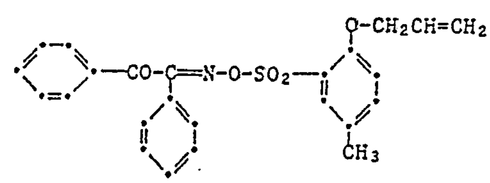

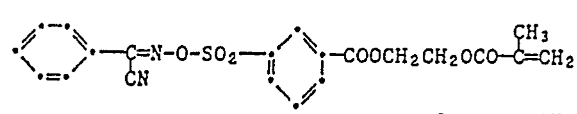

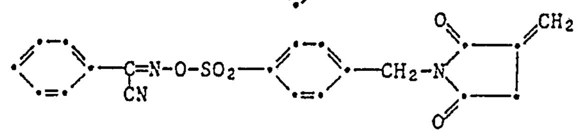

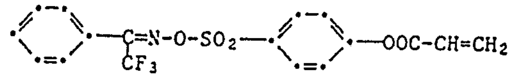

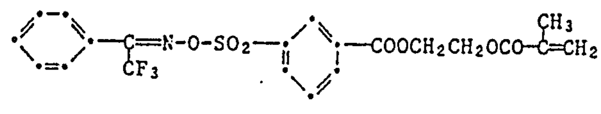

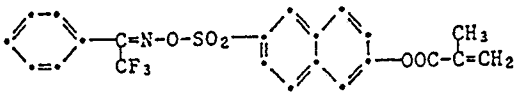

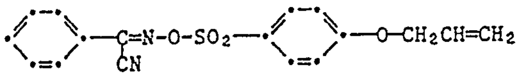

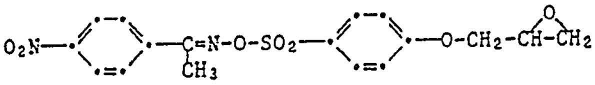

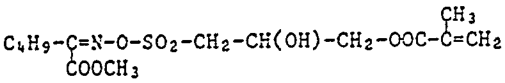

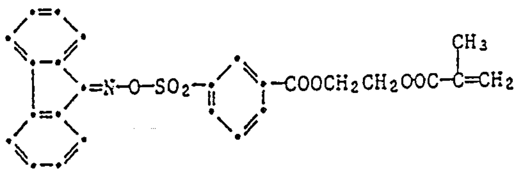

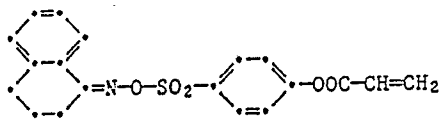

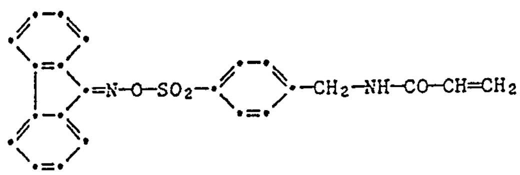

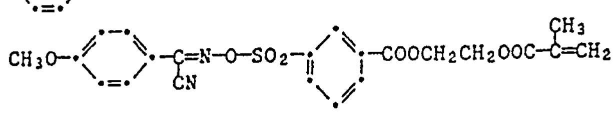

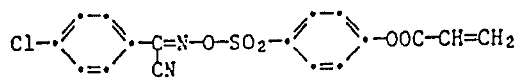

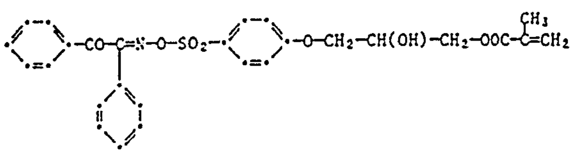

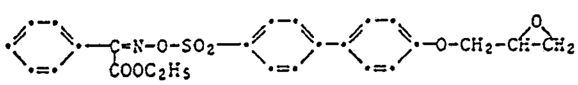

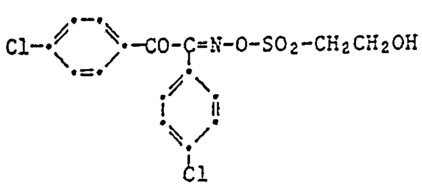

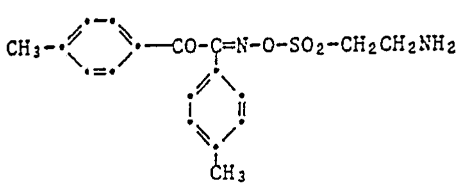

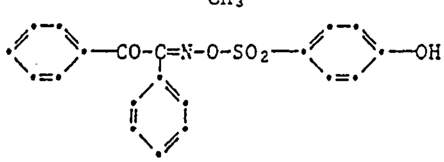

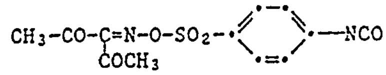

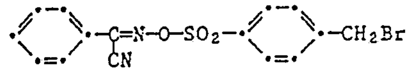

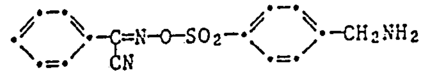

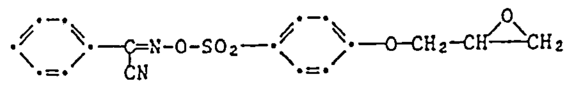

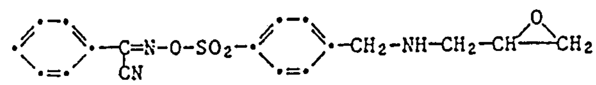

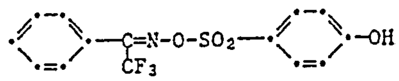

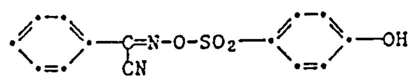

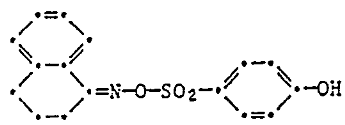

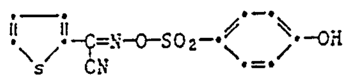

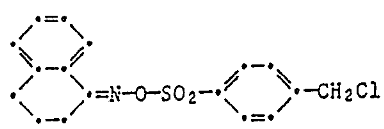

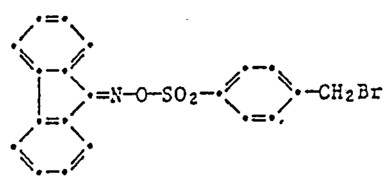

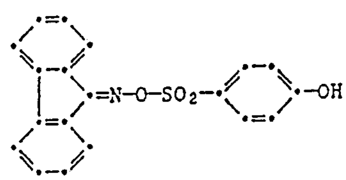

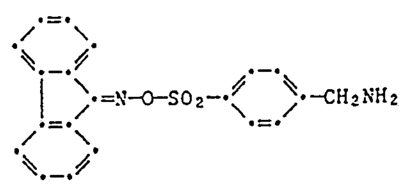

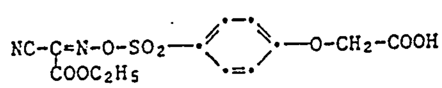

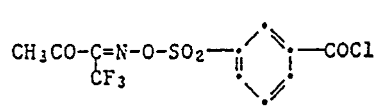

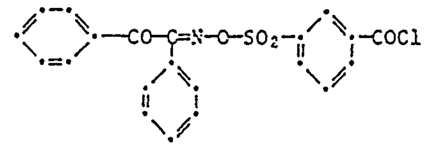

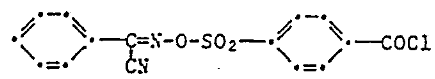

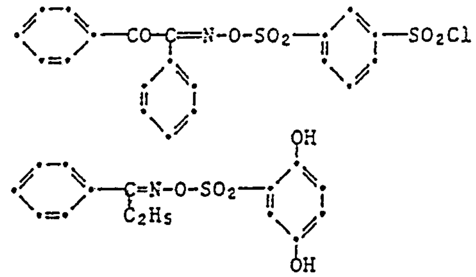

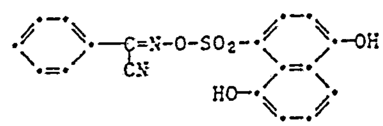

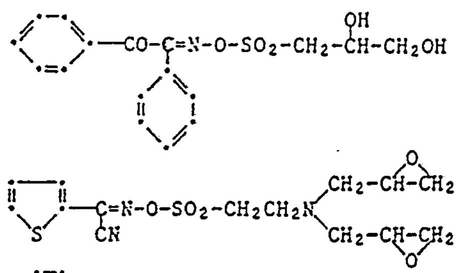

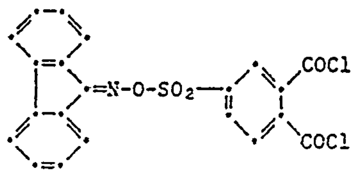

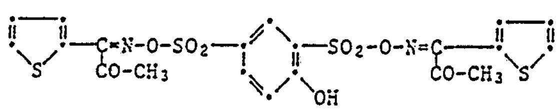

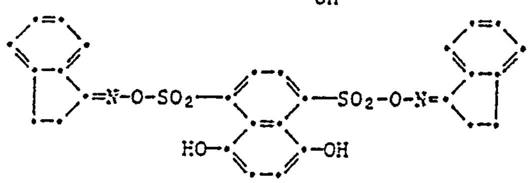

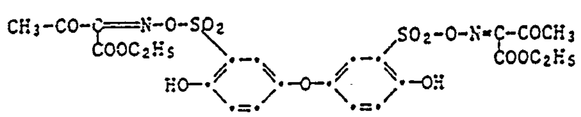

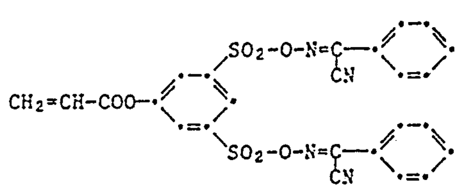

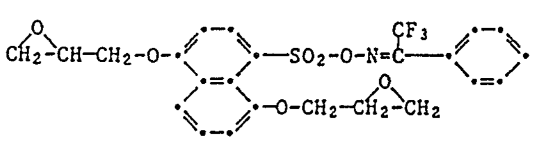

Zur Herstellung der Verbindungen der Formel I gibt es verschiedene Möglichkeiten. Einmal kann man eine Oximverbindung der Formel II mit einem Sulfonsäurechlorid der Formel III umsetzen:

$$x\ R^1-(CO)_m-\underset{\displaystyle R^2}{\underset{|}{C}}=NOH + [ClSO_2]_{\overline{x}}Z-(Y)_y \rightarrow I$$

II III

Man kann diese Umsetzung auch mit einem Sulfochlorid

$$[ClSO_2]_{\overline{x}}Z-(Y')_y$$

machen und führt die Gruppe Y' anschliessend in eine Gruppe Y über. Beispielsweise kann Y' eine OH-Gruppe sein, die anschliessend mit Acryl- oder Methacrylsäure verestert wird oder die anschliessend mit Epichlorhydrin und Alkalihydroxid in den Glycidylether übergeführt wird. Wenn Y' eine $NH_2$-Gruppe ist, so kann diese z. B. mit (Meth)acrylsäurechlorid acyliert werden oder durch Umsetzung mit Epichlorhydrin eine oder zwei Glycidylgruppen eingeführt werden. Wenn Y' eine Carboxylgruppe ist, so kann diese in eine Gruppe $-COCl$ oder $-COOR^6$ überführt werden. Isocyanatgruppen können durch Phosgenierung einer Gruppe $Y'=NH_2$ hergestellt werden. Wenn die Gruppe Y' Halogen ist, so kann dieses in eine Gruppe OH oder $NHR^5$ überführt werden. Die hier aufgeführten Methoden zur Einführung von Y sind nur einige Beispiele aus einer Vielzahl von möglichen Methoden, die man danach auswählen wird, ob Z ein aromatischer oder aliphatischer Rest ist und welche Art von reaktiver Gruppe Y gewünscht ist. Prinzipiell kann die Einführung von Y in den Sulfosäurerest vor oder nach der Sulfonylierung des Oxims geschehen. Es gibt auch eine Reihe von durch Y substituierten Sulfonsäuren, die technische Handelsprodukte sind, wie z. B. Taurin, Isethionsäure, Sulfanilsäure, Metanilsäure, und verschiedene Phenolsulfonsäuren, Naphtholsulfonsäuren und Naphthylaminsulfonsäuren.

Wie bereits erwähnt lassen sich die Verbindungen der Formel I in Polymere überführen, die Seitenketten mit Oximsulfonatgruppen enthalten. Bei Erhitzen oder Belichten, insbesondere bei UV-Bestrahlung, spalten sich die Oximsulfonatgruppen unter Bildung der freien Sulfonsäuren. Es entstehen also Polymere mit Sulfonsäuregruppen. Diese Polymeren sind in wässerigen Alkalien löslich, beispielsweise in $Na_2CO_3$-Lösung oder verdünnter Natronlauge. Die Oximsulfonat-Polymeren sind daher als Photoresists verwendbar, die nach UV-Belichtung mit wässerigen Alkalien zu Positiv-Abbildungen entwickelt werden können. Solche Photoresists werden für die Herstellung gedruckter Schaltungen oder anderer elektronischer Bauelemente benötigt, aber auch für die Herstellung von Druckplatten und für andere photographische Aufzeichnungsverfahren.

Die Erfindung betrifft daher auch Polymere, die in Seitengruppen Reste der Formel IV enthalten.

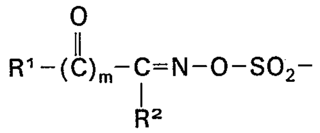 IV

worin m, $R^1$ und $R^2$ die eingangs gegebenen Bedeutungen haben.

Bevorzugt sind solche Polymere, die durch Polymerisation einer Verbindung der Formel I entstehen, worin Y eine ethylenisch ungesättigte Gruppe ist, oder durch Copolymerisation einer solchen Verbindung mit einer anderen ethylenisch ungesättigten Verbindung.

Bevorzugt sind darunter Polymere, die durch Copolymerisation einer Verbindung der Formel I, worin Y eine ethylenisch ungesättigte Gruppe ist, mit einer oder mehreren α,β-ungesättigten Carbonsäuren oder deren Derivaten entstehen, insbesondere durch Copolymerisation mit Acrylsäure, Methacrylsäure oder deren Alkylestern.

Bevorzugt sind weiterhin Polymere, die durch Reaktion eines hydroxylgruppenhaltigen Polymeren mit einer Verbindung der Formel I, worin Y eine Gruppe $-COCl$, $-SO_2Cl$ oder $-NCO$ ist, entstehen.

Die erfindungsgemässen Polymeren können hohe Molekulargewichte von 10000 bis 1000000 haben. Sie können aber auch relativ niedermolekulare Prepolymere sein, die nach Belichtung und Entwicklung in einen hochmolekularen Zustand überführt werden, beispielsweise durch eine Vernetzungsreaktion bei höherer Temperatur.

Zur Belichtung eignen sich Lichtquellen mit hohem Anteil an kurzwelligem Licht. Hierfür stehen heute entsprechende technische Vorrichtungen und verschiedenen Lampenarten zur Verfügung. Beispiele sind Kohlelichtbogenlampen, Xenolichtbogenlampen, Quecksilberdampflampen, Metall-Halogenlampen, Fluoreszenzlampen, Argonglühlampen oder photographische Flutlichtlampen. Neuerdings werden auch Laserlichtquellen verwendet. Diese haben den Vorteil, dass keine Photomasken notwendig sind; der gesteuerte Laserstrahl schreibt direkt auf die Polymer-Schicht.

In bestimmten Fällen ist es von Vorteil, dem Polymeren einen Photosensibilisator zuzusetzen. Dadurch können die Bestrahlungszeiten verkürzt oder andere Lichtquellen benutzt worden. Beispiele für bekannte Photosensibilisatoren sind aromatische Ketone und Aldehyde (wie sie z. B. in der US-A-4.017.652 beschrieben sind), Acylcumarine, Thioxanthone, kondensierte Aromaten wie z. B. Perylen, Anthracen, aromatische Amine oder kationische Farbstoffe, wie sie z. B. in der US-A-4.026.705 beschrieben sind. Solche Sensibilisatoren erhöhen die Empfindlichkeit des Polymeren für die photochemische Erzeugung des latenten Bildes ohne die Lagerstabilität des Polymeren zu verringern. Durch gezielte Auswahl eines Photosensibilisators kann die spektrale Empfindlichkeit des Polymeren in gewünschte Wellenlängenbereiche verschoben werden.

Ausser den Sensibilisatoren können den Oximsulfonat-Polymeren noch andere Additive zugesetzt werden, wie sie für lichtempfindliche Massen üblich sind. Dies sind z. B. Farbstoffe, Pigmente, Antihalationszusätze, polymere Bindemittel, Reaktivverdünner, Adhäsionspromotoren oder Flexibilisatoren.

Die Oximsulfonat-Polymeren sind aber auch für die Erzeugung negativer Abbildungen verwendbar, wenn man die bei Belichtung entstehenden polymeren Sulfosäuren als Härtungskatalysatoren für säurehärtbare Harze verwendet, z. B. für Phenoplaste, Aminoplaste oder Epoxidharze.

Ausser der Verwendung in verschiedenen Bildaufzeichnungsverfahren können die Oximsulfonat-Polymeren in Kombination mit einem pHempfindlichen Farbstoff als Indikatoren zur Kontrolle auf Erhitzung oder Bestrahlung empfindlicher Güter dienen oder für thermoschreibende Systeme.

Sowohl die monomeren Oximsulfonate der Formel I wie auch die daraus hergestellten Polymeren können als latente Säurekatalysatoren zur Härtung von säurehärtbaren Einbrennlacken verwendet werden. Beispiele hierfür sind vor allem Lacke auf Basis von Melaminharzen und deren Gemischen mit Acryl-, Alkyd- und Polyesterharzen. Man setzt solchen Lacken etwa 0,1 bis 10%, bezogen auf das Lackharz, eines Oximsulfonates zu. Nach der Applikation des Lackes wird der Lackfilm kurzzeitig mit kurzwelligem Licht – insbesondere mit UV-Licht – bestrahlt und anschliessend durch Erwärmen ausgehärtet. Vorzugsweise erfolgt das Erwärmen bei 80 bis 120° C.

Die folgenden Beispiele beschreiben im Detail die Herstellung der Monomeren, ihre Überführung in Polymere und deren Verwendung. Darin bedeuten Teile Gewichtsteile.

*Beispiel 1:*

*α-(3-[2-(Methacryloxy)-ethoxycarbonyl]-benzolsulfonyloxyimino)-benzylcyanid*

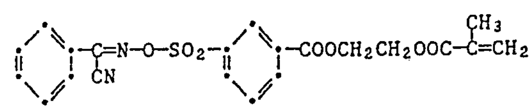

1a) *3-Chlorsulfonyl-benzoylchlorid*

Die Verbindung wird nach der in der US-A-3,290,370 beschriebenen Methode durch Chlorsulfonierung von α-Trichlortoluol erhalten.

1b) *3-[2-(Methacryloxy)-ethoxycarbonyl]-benzolsulfochlorid*

Zu einer Lösung von 23,9 g (0,1 Mol) 3-Chlorsulfonyl-benzoylchlorid und 7,9 g (0,1 Mol) Pyridin in 150 ml Tetrahydrofuran werden unter Rühren bei 0° C eine Lösung von 13 g (0,1 Mol) 2-Hydroxyethyl-methacrylat in 80 ml THF langsam zugetropft. Anschliessend wird 15 h bei Raumtemperatur gerührt. Das ausgefallene Pyridinsalz wird abfiltriert und das Filtrat im Vakuum eingedampft. Der ölige Rückstand wird im Hochvakuum getrocknet. Man erhält 28 g viskoses Produkt.

*Analyse* $C_{13}H_{13}ClO_6S$ (332,74):

| | | | | |
|---|---|---|---|---|
| Ber. | C 46,92 | H 3,94 | S 9,63 | Cl 10,65% |
| Gef. | C 47,40 | H 4,04 | S 9,49 | Cl 10,36% |

1c) *α-Hydroxyimino-benzylcyanid*

Die Verbindung wird hergestellt nach der in Org. Synthesis *59*, 95 (1979) beschriebenen Methode aus Benzylcyanid durch Reaktion mit Methylnitrit in $CH_3OH/CH_3ONa$. Farblose Kristalle vom Smp. 119-121° C.

1d) *α-(3-[2-(Methacryloxy)-ethoxycarbonyl]-benzolsulfonyloxyimino)-benzylcyanid*

Zu einer Lösung von 7,3 g (0,05 Mol) α-Hydroxyimino-benzylcyanid (1c) und 5,05 g (0,05 Mol) Triethylamin in 100 ml Methylenchlorid wird unter Kühlung auf 0° C eine Lösung von 16,6 g (0,05 Mol) 3-[2-(Methacryloxy)-ethoxycarbonyl]-benzolsulfochlorid (1b) in 50 ml $CH_2Cl_2$ unter Rühren zugetropft. Anschliessend wird 18 h bei Raumtemperatur gerührt. Das Reaktionsgemisch wird mit Wasser versetzt, die organische Phase abgetrennt, mit Wasser gewaschen und über $MgSO_4$ getrocknet. Die Lösung wird eingedampft und der ölige Rückstand im Hochvakuum getrocknet. Man erhält 14,5 g der Titelverbindung als viskose farblose Masse.

*Analyse* $C_{21}H_{18}N_2O_7S$ (442,41):

| | | | | |
|---|---|---|---|---|
| Ber. | C 57,01 | H 4,10 | N 6,33 | S 7,25% |
| Gef. | C 56,92 | H 4,18 | N 6,26 | S 7,03% |

$^1H$-NMR (100 MHz, $CDCl_3$): 8,75 (t, J=2, 1 aromat. H), 8,5-8,2 (m, 2 aromat. H), 7,9-7,35 (m, 6 aromat. H), 6,18 (s mit FS, 1 vinylisches H), 5,60 (t, J=2, 1 vinylisches H), 4,75-4,4 (m, 2 $-CH_2O-$) 1,95 (t, J=2, $CH_3$)

IR-Spektrum: 2235w (C≡N), 1720s (CO-Ester), 1630w (C=C), 1264m und 1195s ($SO_2OR$).

*Beispiel 2:*

*9-(3-[2-(Methacryloxy)-ethoxycarbonyl]-benzolsulfonyloxyimino)-fluoren*

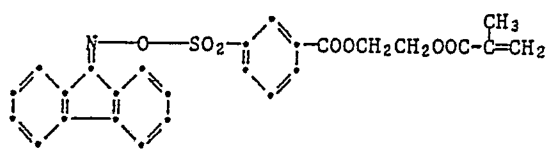

Die Verbindung wird analog Beispiel 1d aus Fluorenonoxim und 1b in Tetrahydrofuran hergestellt.

Das Rohprodukt ist eine gelbe hochviskose Masse.

*Analyse* $C_{26}H_{21}NO_7S$ (491,7):

| | | | | |
|---|---|---|---|---|
| Ber. | C 63,54 | H 4,31 | N 2,85 | S 6,52% |
| Gef. | C 63,92 | H 4,31 | N 3,02 | S 6,32% |

IR-Spektrum: 1720s, 1630w, 1261m, 1190s.

Das $^1H$-NMR-Spektrum stimmt mit der angegebenen Struktur überein.

*Beispiel 3:*

*α-(4-Hydroxybenzolsulfonyloxyimino)-thien-2-ylacetonitril*

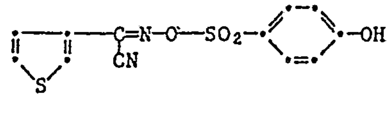

3a) *4-Acetoxy-benzolsulfonsäurechlorid*

270 g einer 65%igen Lösung von Phenol-4-sulfonsäure in Wasser (1 Mol) werden mit 100 ml Toluol zu einer Suspension verrührt. Unter kräftigem Rühren werden erst 113 g KOH (2 Mol) in 240 ml Wasser zugegeben und dann 110 g (1 Mol) Essigsäureanhydrid zugetropft. Anschliessend wird 15 h bei Raumtemperatur gerührt. Das Reaktionsgemisch wird mit 300 ml Wasser versetzt, die wässerige Phase abgetrennt und im Vakuum eingedampft. Das hinterbliebene Kaliumsalz der 4-Acetoxybenzolsulfonsäure wird mit heissem Ethanol gewaschen und getrocknet.

254 g (1 Mol) des so erhaltenen Salzes wird portionenweise in eine auf 0° C gekühlte Lösung von 416 g (2 Mol) $PCl_5$ in 1 l $CCl_4$ eingetragen und anschliessend 16 h bei Raumtemperatur gerührt. Das Reaktionsgemisch wird auf Eis gegossen, die organische Phase abgetrennt, über $MgSO_4$ getrocknet und eingedampft. Das hinterbleibende Rohprodukt wird aus Diethylether umkristallisiert. Smp. 76-78° C.

*Analyse* $C_8H_7ClO_4S$ (234,66):

| | | | | |
|---|---|---|---|---|
| Ber. | C 40,95 | H 3,01 | Cl 15,11 | S 13,66% |
| Gef. | C 40,71 | H 2,94 | Cl 15,15 | S 13,67% |

3b) *α-Hydroxyimino-thien-2-ylacetonitril*

Die Verbindung wird analog der in Org. Synthesis *59*, 95 (1979) beschriebenen Methode aus Thiophen-2-acetonitril und Methylnitrit hergestellt. Gelbliche Kristalle vom Smp. 103-104° C.

*Analyse* $C_6H_4N_2OS$ (152,06):

| | | | | |
|---|---|---|---|---|
| Ber. | C 47,36 | H 2,65 | N 18,41 | S 21,07% |
| Gef. | C 47,25 | H 2,79 | N 18,21 | S 21,14% |

3c) *α-(4-Acetoxybenzolsulfonyloxyimino)-thien-2-ylacetonitril*

Eine Lösung von 12 g (0,08 Mol) α-Hydroxyimino-thien-2-yl-acetonitril (3b) in 75 ml Tetrahydrofuran wird mit 200 mg 4-Dimethylaminopyridin und 6,2 g (0,08 Mol) Pyridin versetzt. Unter Kühlung auf 0° C wird eine Lösung von 18,5 g (0,08 Mol) 4-Acetoxy-benzolsulfochlorid (3a) in 30 ml Tetrahydrofuran zugetropft. Das Reaktionsgemisch wird 16 h bei Raumtemperatur gerührt und anschliessend mit 100 ml Diethylether versetzt. Die ausgefallenen Salze werden abfiltriert und die Lösung eingedampft. Das zurückbleibende Rohprodukt wird aus Hexan/Ethylacetat umkristallisiert. Man erhält 15 g Kristalle vom Smp. 114-115° C.

*Analyse* $C_{14}H_{10}N_2O_5S_2$ (350,34):

| | | | |
|---|---|---|---|
| Ber. | C 47,99 | H 2,88 | N 8,00% |
| Gef. | C 47,71 | H 2,94 | N 8,35% |

3d) *α-(4-Hydroxybenzolsulfonyloxyimino)-thien-2-ylacetonitril*

Eine Lösung von 27 g (0,08 Mol) α-(4-Acetoxybenzolsulfonyloxyimino)-thienylacetonitril (3c) in 125 ml Tetrahydrofuran wird unter kräftigem Rühren mit einer Lösung von 12 g NaOH in 20 ml Wasser versetzt. Nach einer Stunde wird mit 20%iger Salzsäure angesäuert und nach Zugabe

von Wasser und Diethylether werden die Phasen getrennt. Die Wasserphase wird mit Ether extrahiert, die vereinigten organischen Phasen über $MgSO_4$ getrocknet und das Lösungsmittel abdestilliert. Das hinterbleibende Rohprodukt wird aus Toluol umkristallisiert. Man erhält 17 g beige Kristalle, die bei 151-152° C schmelzen.

*Analyse* $C_{12}H_8N_2O_4S_2$ (308,3):

| | | | | |
|---|---|---|---|---|
| Ber. | C 46,75 | H 2,62 | N 9,01 | S 20,80% |
| Gef. | C 47,19 | H 2,70 | N 8,94 | S 20,27% |

IR-Spektrum: 3400 und 3300br(OH), 2225s (CN), 1375s ($SO_2$)

$^1$H-NMR-Spektrum (100 MHz, $CDCl_3$): 7,96 (d, 2H),

8,05-7,83 (m, 1H), 7,7-7,55 (m, 1H), 7,25-6,9 (m, 1H), 7,02 (d, 2H), 1,8 (s, OH).

*Beispiel 4:*

*α-(4-Methacryloxy-benzolsulfonyloxyimino)-thien-2-ylacetonitril*

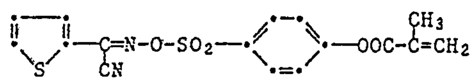

Eine Lösung von 6,1 g (0,022 Mol) α-(4-Hydroxybenzolsulfonyloxyimino)-thien-2-ylacetonitril (3a) in 75 ml Methylenchlorid wird mit 2,2 g (0,022 Mol) Triethylamin und 0,5 g 4-Dimethylamino-pyridin versetzt. Unter Kühlung auf 0° C wird eine Lösung von 2,3 g (0,022 Mol) Methacrylsäurechlorid in 20 ml $CH_2Cl_2$ zugetropft und das Reaktionsgemisch 3 h bei 0° C gerührt. Danach wird über Kieselgel filtriert, das Filtrat eingedampft und der Rückstand aus Methylenchlorid/-Hexan umkristallisiert. Man erhält 5,92 g vom Smp. 165-166° C.

*Analyse* $C_{16}H_{12}N_2O_5S_2$ (376,38):

| | | | | |
|---|---|---|---|---|
| Ber. | C 51,06 | H 3,22 | N 7,44 | S 17,04% |
| Gef. | C 50,76 | H 3,05 | N 7,26 | S 17,07% |

$^1$H-NMR-Spektrum und IR-Spektrum stimmen mit der angegebenen Struktur überein.

*Beispiel 5:*

*α-(4-Hydroxybenzolsulfonyloxyimino)-benzylcyanid*

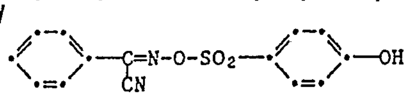

14,6 g (0,1 Mol) α-Hydroxyimino-benzylcyanid (1c) werden mit 23,46 g (0,1 Mol) 4-Acetoxybenzolsulfochlorid (3a) analog zu Beispiel 3c umgesetzt. Das erhaltene α-(4-Acetoxybenzolsulfonyloxyimino)-benzylcyanid schmilzt bei 142-143°C.

6 g dieser Verbindung werden analog Beispiel 3d mit Natronlauge hydrolysiert und man erhält die Titelverbindung, die nach Umkristallisation aus Toluol bei 148° C schmilzt.

*Analyse* $C_{14}H_{10}N_2O_4S$ (302,3):

| | | | | |
|---|---|---|---|---|
| Ber. | C 55,63 | H 3,34 | N 9,27 | S 10,61% |
| Gef. | C 55,53 | H 3,55 | N 8,89 | S 10,39% |

IR-Spektrum: 3415s (OH), 2235 W (CN), 1368s und 1182s ($SO_2$).

Das $^1$H-NMR-Spektrum stimmt mit der angegebenen Struktur überein.

*Beispiel 6:*

*1-(4-Hydroxybenzolsulfonyloxyimino)-1,2,3,4-tetrahydronaphthalin*

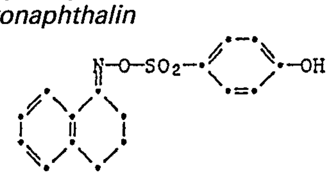

6a) *α-Tetralonoxim*

Die Verbindung wird nach der in Chem. Ber. *54*, 57 (1921) beschriebenen Methode aus α-Tetralon und $NH_2OH \cdot HCl$ in Gegenwart von $K_2CO_3$ in Methanol hergestellt. Smp. 101-104° C.

6b) *1-(4-Acetoxybenzolsulfonyloxyimino)-1,2,3,4-tetrahydronaphthalin*

Die Verbindung wird analog Beispiel 3c aus α-Tetralonoxim (6a) und 4-Acetoxybenzolsulfochlorid (3a) hergestellt. Smp. 151-152° C.

*Analyse* $C_{18}H_{17}NO_5S$ (359,38):

| | | | | |
|---|---|---|---|---|
| Ber. | C 60,16 | H 4,77 | N 3,90 | S 8,92% |
| Gef. | C 59,94 | H 4,99 | N 3,99 | S 8,92% |

6c) *1-(4-Hydroxbenzolsulfonyloxyimino)-1,2,3,4-tetrahydronaphthalin*

Aus obiger Verbindung (6b) durch alkalische Hydrolyse analog Beispiel 3d. Farblose Kristalle vom Smp. 141-142° C.

*Analyse* $C_{16}H_{15}NO_4S$ (317,34):

| | | | | |
|---|---|---|---|---|
| Ber. | C 60,55 | H 4,76 | N 4,41 | S 10,10% |
| Gef. | C 60,49 | H 4,88 | N 4,37 | S 10,17% |

IR-Spektrum: 3375s, 1348s, 1174s.

Das $^1$H-NMR-Spektrum stimmt mit der angegebenen Struktur überein.

*Beispiel 7:*

*1-(4-Methacryloxybenzolsulfonyloxyimino)-1,2,3,4-tetrahydronaphthalin*

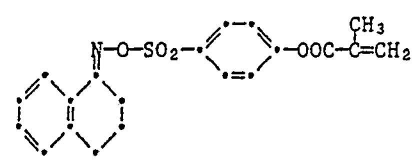

Das Produkt aus Beispiel 6c wird analog Beispiel 4 mit Methacrylsäurechlorid umgesetzt. Farblose Kristalle vom Smp. 108-109° C.

*Analyse* $C_{20}H_{19}NO_5S$ (385,41):

| | | | | |
|---|---|---|---|---|
| Ber. | C 62,32 | H 4,97 | N 3,63 | S 8,32% |
| Gef. | C 62,11 | H 5,05 | N 3,53 | S 8,30% |

IR-Spektrum: 1724s, 1622w, 1373s, 1182s.

Das $^1$H-NMR-Spektrum stimmt mit der angegebenen Struktur überein.

*Beispiel 8:*

*α-Trifluoracetophenonoxim-4-hydroxybenzolsulfonat*

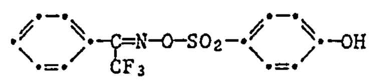

8a) *α-Trifluoracetophenonoxim*

Die Verbindung wird analog Beispiel 6a aus Trifluoracetophenon und Hydroxylamin hergestellt.

8b) *α-Trifluoracetophenonoxim-4-acetoxybenzolsulfonat*

Die Verbindung wird aus 8a durch Umsetzung mit 4-Acetoxybenzolsulfochlorid (3a) analog Beispiel 3c hergestellt. Das ölige Rohprodukt wird ohne weitere Reinigung für die nächste Reaktionsstufe verwendet.

8c) *α-Trifluoracetophenonoxim-4-hydroxybenzolsulfonat*

Die Acetoxyverbindung 8b wird analog Beispiel 3d mit NaOH hydrolysiert. Farblose Kristalle vom Smp. 120-121° C.

*Analyse* $C_{14}H_{10}F_3NO_4S$ (345,25):

| | | | | | |
|---|---|---|---|---|---|
| Ber. | C 48,70 | H 2,92 | N 4,06 | S 9,28 | F 16,50% |
| Gef. | C 48,63 | H 2,87 | N 3,97 | S 9,47 | F 16,64% |

IR-Spektrum: 3385s (OH), 1178s ($SO_2$), 1160, 1155 und 1143s (C-F), 548s (C-F).

$^1$H-NMR-Spektrum (100 MHz, $CDCl_3$): 7,90 (d, I=8,75, 2 aromat. H), 7,60-7,30 (m, 5 aromat. H), 7,01 (d, I=8,75, 2 aromat. H), 5,98 (s, OH).

*Beispiel 9:*

*9-(4-Hydroxybenzolsulfonyloxyimino)-fluoren*

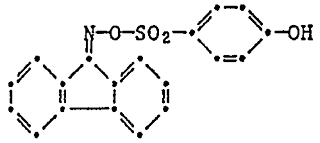

Fluorenonoxim wird analog Beispiel 3c mit 4-Acetoxybenzolsulfochlorid (3a) umgesetzt zum 9-(4-Acetoxybenzolsulfonyloxyimino)-fluoren, das bei 167-168° C schmilzt.

Diese Verbindung wird mit NaOH hydrolysiert analog Beispiel 3d. Man erhält die Titelverbindung in Form von gelblichen Kristallen vom Smp. 216-217° C.

*Analyse* $C_{19}H_{13}NO_4S$ (351,4):

| | | | | |
|---|---|---|---|---|
| Ber. | C 64,95 | H 3,73 | N 3,99 | S 9,12% |
| Gef. | C 65,10 | H 3,97 | N 3,93 | S 8,60% |

IR-Spektrum: 3415s, 1370s, 1180s.

Das $^1$H-NMR-Spektrum stimmt mit der angegebenen Struktur überein.

*Beispiel 10:*

*9-(4-Brommethyl-benzolsulfonyloxyimino)-fluoren*

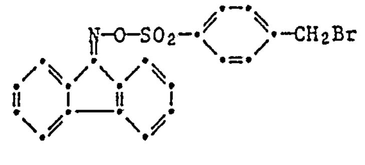

Fluorenonoxim (hergestellt nach der Methode von F.J. Moore und E.H. Hunters/J. Amer. Chem. Soc. *49* (1927), 2618) wird bei 0° in Tetrahydrofuran in Gegenwart von einem Äquivalent Triethylamin mit einem Äquivalent p-Toluolsulfochlorid umgesetzt. Nach Aufarbeitung analog Beispiel 3c erhält man das Fluorenonoxim-tosylat das bei 155-160° C schmilzt.

*Analyse:*

Ber. 9,15% S,

Gef. 9,18% S.

Zur Lösung von 144 g (0,41 Mol) des Tosylates in 1500 ml $CCl_4$ werden 73,3 g (0,41 Mol) N-Bromsuccinimid und 3 g Azobis-isobutyronitril zugegeben. Die Suspension wird 4 h zum Rückfluss erwärmt. Nach dem Abkühlen wird das Succinimid abfiltriert und das Filtrat eingedampft. Das hinterbleibende rohe Monobromid ist durch Dibromid und Ausgangsmaterial verunreinigt. Durch wiederholte Umkristallisation aus Hexan/Tetrahydrofuran erhält man das reine Monobromid, das bei 151-152° C schmilzt.

*Analyse* $C_{20}H_{14}BrNO_3S$ (428,3):

| | | | | | |
|---|---|---|---|---|---|
| Ber. | C 56,09 | H 3,29 | N 3,27 | S 7,49 | Br 18,66% |
| Gef. | C 55,86 | H 3,32 | N 3,32 | S 7,51 | Br 18,98% |

IR-Spektrum: 1368s, 1185s.

$^1$H-NMR-Spektrum (100 MHz, $CDCl_3$): 8,35-8,0 m, 7,75-7,1 m, 4,5s.

*Beispiel 11:*

*α-(4-Brommethyl-benzolsulfonyloxyimino)-benzylcyanid*

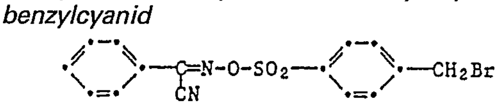

α-Tosyloxyiminobenzylcyanid wird analog Beispiel 1d aus α-Hydroxyiminobenzylcyanid und p-Toluolsulfochlorid hergestellt.

Smp. 179-181° C.

*Analyse:*

Ber. 10,67%S

Gef. 10,67%S.

Die Verbindung wird analog Beispiel 10 mit Bromsuccinimid in Tetrachlormethan bromiert. Das nach Umkristallisation aus Hexan/Tetrahydrofuran erhaltene Monobromid schmilzt bei 171-172° C.

*Analyse* $C_{15}H_{11}BrN_2O_3S$ (379,20):

| | | | | | |
|---|---|---|---|---|---|
| Ber. | C 47,51 | H 2,92 | N 7,39 | S 8,45 | Br 21,07% |
| Gef. | C 47,62 | H 2,98 | N 7,45 | S 8,70 | Br 20,88% |

IR-Spektrum: 2225w, 1378s, 1188s.

$^1$H-NMR-Spektrum: 8,05d, 7,9-7,35m, 7,64d, 4,51s.

*Beispiel 12:*

*9-(4-Aminomethyl-benzolsulfonyloxyimino)-fluoren*

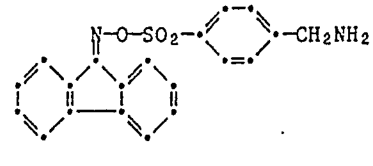

Eine Lösung von 85,7 g (0,2 Mol) 9-(4-Brommethyl-benzolsulfonyl-oxyimino)-fluoren (Beispiel 10) in 500 ml Chloroform wird mit einer Lösung von 28 g (0,2 Mol) Hexamethylentetramin in 350 ml $CHCl_3$ versetzt und 1 h bei Raumtemperatur gerührt. Das ausgefallene Salz wird abfiltriert und mit $CHCl_3$ gewaschen. Smp. 196-197° C.

*Analyse:*

| | |
|---|---|
| Ber. | Br 14,0% |
| Gef. | Br 13,7% |

56,8 g (0,1 Mol) des Salzes werden in einer Mischung von 88 ml Wasser und 88 ml Ethanol suspendiert und mit 42 ml konzentrierter Salzsäure versetzt. Das Reaktionsgemisch wird 12 h bei Raumtemperatur und 1 h bei 50° C gerührt. Nach dem Abkühlen wird NaOH-Lösung bis zum pH 8 zugegeben und mit Methylenchlorid extrahiert. Die organische Phase wird getrocknet und eingedampft, der Rückstand aus Ethanol umkristallisiert. Gelbliche Kristalle vom Smp. 106-107° C.

*Analyse* $C_{20}H_{16}N_2O_3S$ (364,4):

| | | | | |
|---|---|---|---|---|
| Ber. | C 65,92 | H 4,43 | N 7,69 | S 8,80% |
| Gef. | C 66,22 | H 4,66 | N 7,14 | S 8,71% |

IR-Spektrum: 3400br, 1370s, 1185s.

$^1$H-NMR-Spektrum: 8,29d, 8,05d, 7,70-7,10m, 3,69s, 3,4s, 2,15s.

*Beispiel 13:*

*9-(4-Methacrylamidomethyl-benzolsulfonyl-oxyimino)-fluoren*

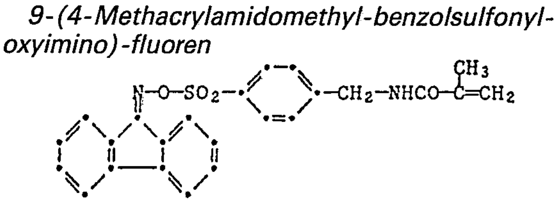

In eine Lösung von 5 g (0,013 Mol) des Produktes von Beispiel 12 und 2,77 g (0,026 Mol) Triethylamin in 50 ml Tetrahydrofuran wird bei 0° C eine Lösung von 2,87 g (0,026 Mol) Methacrylsäurechlorid zugetropft. Das Reaktionsgemisch wird 12 h gerührt und dann mit Wasser und Diethylether versetzt. Die organische Phase wird mit verdünnter Salzsäure gewaschen, über $MgSO_4$ getrocknet und eingedampft. Das ölige Rohprodukt wird aus Hexan/Ethylacetat kristallisiert. Gelbliche Kristalle vom Smp. 187-188° C.

*Analyse* $C_{24}H_{20}N_2SO_4$ (432,46):

| | | | |
|---|---|---|---|
| Ber. | C 66,65 | H 4,66 | S 7,41% |
| Gef. | C 66,49 | H 5,13 | S 6,90% |

IR-Spektrum und $^1$H-NMR-Spektrum stimmen mit der angegebenen Struktur überein.

*Beispiel 14:*

*α-(4-Carboxy-benzolsulfonyloxyimino)-benzylcyanid*

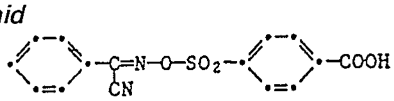

5 g α-Hydroxyiminobenzylcyanid werden in 24 g Tetrahydrofuran gelöst und bei Temperaturen zwischen −10° C und 0° C unter Rühren langsam mit einer Lösung von 7,55 g p-Chlorsulfonylbenzoesäure in 25 g Tetrahydrofuran versetzt. Danach lässt man unter Rühren auf Raumtemperatur erwärmen und giesst nach 2 h die Reaktionsmischung in 600 ml 0,12 N Salzsäure. Das ausgefallene Produkt wird abfiltriert, mit Wasser gewaschen und bei 40° C im Vakuum getrocknet.

Smp. 250-251° C. IR-Spektrum und $^1$H-NMR-Spektrum stimmen mit der angegebenen Struktur überein.

*Beispiel 15:*

*α-(4-Chlorcarbonyl-benzolsulfonyloxyimino)-benzylcyanid*

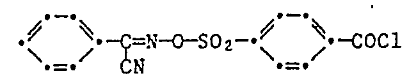

4 g des Produktes von Beispiel 14 werden in 20 g Thionylchlorid suspendiert und mit 0,5 ml Dimethylformamid versetzt. Man heizt danach auf 50° C bis die Gasentwicklung aufhört (2 h) und eine klare Lösung entstanden ist. Man dampft das überschüssige Thionylchlorid teilweise ab und erhält ein kristallines Produkt, das abfiltriert und aus Dichlormethan umkristallisiert wird. Smp. 193-195° C.

*Beispiel 16-21:*

*Herstellung von Copolymerisaten*

Verbindungen der Formel I, worin Y eine ethylenisch ungesättigte Gruppe ist, werden mit verschiedenen Comonomeren in Methylethylketon als Lösungsmittel unter Stickstoff polymerisiert. Die Reaktionsbedingungen sind in Tabelle 1 aufgeführt. Die nach Fällung mit Hexan erhaltenen Polymeren sind weisse bis leicht gelbe Pulver, die in vielen gebräuchlichen organischen Lösungsmitteln wie z. B. Tetrahydrofuran, Ethylglykolacetat, Ethylcellosolve, Aceton etc. löslich sind und zu klaren Filmen verarbeitet werden können. Tabelle 1 zeigt die Zusammensetzung, Herstellungsbedingungen und Eigenschaften der Copolymeren.

*(Tabelle auf den nächsten Seiten)*

*Beispiel 22-24:*

*Erzeugung von Positivbildern aus Copolymeren*

Von den Copolymeren gemäss Beispielen 16-21 stellt man 20%ige Lösungen in Ethylglykolacetat her und versetzt sie mit 0,2 Gew.-% (bezogen auf Copolymergewicht) Orasolrot B und 2 Gew.-% 9-Methylanthracen. Mit diesen Lösungen beschichtet man mittels eines 20 µm Rakels transparente Polyesterfolien und trocknet die Schicht während 3 min bei 100° C. Es resultieren harte Polymerschichten von ca. 3 µm Dicke. Die Folien werden anschliessend durch eine Maske mit Strichmuster (Strichbreite 0,5 µm bis 5 µm) unter einer Quecksilberhochdrucklampe belichtet und anschliessend in einem wässerig-alkalischen Entwickler während 1-2 min entwickelt. Es resultieren Positivbilder mit einer Auflösung von besser als 1 µm.

Eine Lösung enthaltend Copolymer 16 wird wie oben beschrieben verarbeitet und die Schicht 30 s

belichtet. Nach 2 min Entwicklung in einer Mischung aus 200 ml Positiv-Entwickler C 1290 (Horsell Graphic Industries, Morley, England), 10 g Natriumhydroxyd und 50 ml 2-Ethoxyethanol wird unter leichtem Reiben mit einem Wattebausch ein Positivbild mit einer Auflösung von 1 µm erhalten.

Wie vorstehend beschrieben wird eine Schicht aus dem Copolymeren 18 hergestellt und durch die Maske 30 s und 60 s belichtet. Die Entwicklung erfolgt während 2 min unter Reiben mit einem Wattebausch in einem basischen Entwickler aus 100 ml 1-Methoxy-2-propanol, 50 ml Positiv-Entwickler C 1290 und 5 g NaOH. Nur das 60 s belichtete Muster ist voll entwickelt, zeigt aber ein sehr gutes Positivbild mit einer Auflösung von besser als 1 µm.

*Beispiel 25:*

*Herstellung eines modifizierten Novolak-Harzes*

3 g Novolak-Harz (hergestellt aus Phenol, p-t-Butylphenol und Formaldehyd im Molverhältnis 0,75:0,25:0,90, Erweichungspunkt 120° C) werden in 12 g Pyridin gelöst und bei Raumtemperatur mit 1 g des Säurechlorids aus Beispiel 15 versetzt. Nach 2 h Rühren giesst man die Mischung in 600 ml 0,2 N Salzsäure, filtriert das Festprodukt ab und trocknet. Man löst das Produkt anschliessend in Aceton und fällt erneut in 600 ml 0,02 N Salzsäure, filtriert, wäscht mit Wasser und trocknet im Vakuum bei 40° C.

*Beispiel 26:*

*Erzeugung eines Positivbildes aus modifiziertem Novolak*

1 g des modifizierten Novolaks gemäss Beispiel 25 wird in 2,3 ml eines Lösungsmittelgemisches aus 2-Ethoxyethanol, 2-Ethoxyethylacetat und Ethylmethylketon (Volumenverhältnis 2:2:1) gelöst, mit 2 Tropfen einer Lösung von Kristallviolett im gleichen Lösungsmittelgemisch versetzt und anschliessend auf eine gereinigte, mit Kupfer kaschierte Laminatplatte als Film aufgetragen. Nach 5 min Trocknung bei 90° C resultiert eine 5 µm dikke Lackschicht, die durch ein Negativ mit einer 5000 W Metallhalogenid-Lampe im Abstand von 75 cm während 1 min belichtet wird. Entwicklung in 10%iger wässeriger Natriumkarbonatlösung während 2 min löst die belichteten Stellen heraus und gibt ein gutes positives Bild.

Tabelle 1: *Copolymerisation ethylenisch ungesättigter Oximsulfonate*

| Beispiel | 16 | 17 | 18 | 19 | 20 | 21 |
|---|---|---|---|---|---|---|
| Monomere*) | | | | | | |
| MMA (g) | 40,0 | 7,5 | 40,0 | 16,0 | 16,0 | 7,0 |
| MAS (g) | 5,0 | 7,5 | — | 2,0 | — | 1,0 |
| EHMA (g) | — | 27,5 | — | — | — | — |
| Oximsulfonat (g) | | | | | | |
| aus Beispiel 1 | — | — | — | 2,0 | 4,0 | — |
| Beispiel 2 | 10,0 | 15,0 | 20,0 | — | — | — |
| Beispiel 7 | — | — | — | — | — | 2,0 |
| Polymerisations-bedingungen*) | | | | | | |
| MEK (ml) | 150 | 150 | 150 | 100 | 100 | 50 |
| AIBN (g) | 0,5 | 0,52 | 0,48 | 0,20 | 0,20 | 0,008 |
| Temp. (°C) | 80 | 80 | 80 | 83 | 80 | 83 |
| Zeit (h) | 10 | 10 | 10 | 7,5 | 8,0 | 24 |
| Ausbeute (g) | 54,8 | 50,4 | 54,8 | 16,3 | 16,3 | 9,6 |
| Charakterisierung der Copolymeren*) | | | | | | |
| S-Gehalt (%) | 1,03 | 1,54 | 1,86 | 0,70 | 1,45 | 1,49 |
| $M_w$ (GPC) | 22500 | 37200 | 26400 | 15400 | 13800 | 6700 |

*) Legende

| | |
|---|---|
| MMA | Methylmethacrylat |
| MAS | Methacrylsäure |
| EHMA | 2-Ethylhexylmethacrylat |
| AIBN | α,α'-Azobis-isobutyronitril |
| MEK | Methylethylketon |
| $M_w$ (GPC) | mittleres Molekulargewicht (Gewichtsmittel) bestimmt durch Gelpermeationschromatographie in Tetrahydrofuran |

**Patentansprüche**

1. Verbindungen der Formel I

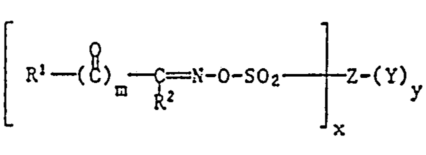

(I)

worin m=0 oder 1, x=1 oder 2 und y=1 oder 2 ist,

$R^1$ $C_1$-$C_{12}$-Alkyl, $C_1$-$C_4$-Halogenalkyl, $C_5$-$C_{12}$-Cycloalkyl, unsubstituiertes oder durch einen oder mehrere der Substituenten Halogen, $C_1$-$C_{16}$-Alkyl, $C_1$-$C_4$-Alkoxy, Phenoxy, Phenyl oder Nitro substituiertes $C_6$-$C_{10}$-Aryl, Furyl, Thienyl, $C_7$-$C_{12}$-Aralkyl, $C_1$-$C_8$-Alkoxy, $C_5$-$C_8$-Cycloalkoxy, Phenoxy oder CN bedeutet,

$R^2$ eine der für $R^1$ gegebenen Bedeutungen hat oder $C_2$-$C_6$-Alkanoyl, Benzoyl, $C_2$-$C_5$-Alkoxycarbonyl, Phenoxycarbonyl, $-N(R^3)(R^4)$, Morpholino oder Piperidino bedeutet oder $R^1$ und $R^2$ zusammen mit den Atomen, an die sie gebunden sind, einen 5-8-gliedrigen Ring bilden, der durch 1 oder 2 Benzoreste anneliert sein kann, wobei $R^3$ Wasserstoff, $C_1$-$C_{12}$-Alkyl, Phenyl, $C_2$-$C_6$-Alkanoyl oder Benzoyl und $R^4$ Wasserstoff, $C_1$-$C_{12}$-Alkyl oder Cyclohexyl bedeuten,

Y eine polymerisierbare ethylenisch ungesättigte Gruppe oder Epoxidgruppe oder eine Gruppe $-OH$, $-NHR^5$, $-COOH$, $-COOR^6$, $-COCl$, $-CH_2Hal$, $-SO_2Cl$ oder $-NCO$ ist, worin $R^5$ Wasserstoff, $C_1$-$C_6$-Alkyl oder Phenyl bedeutet, $R^6$ $C_1$-$C_6$-Alkyl oder Phenyl bedeutet und Hal Chlor oder Brom bedeutet, und

Z ein (x+y)-wertiges organisches Zwischenglied ist, das den Sulforest $-SO_2-$ mit der Gruppe Y verbindet.

2. Verbindungen gemäss Anspruch 1 der Formel I, worin Z eine (x+y)-wertige aromatische, aliphatische, cycloaliphatische oder araliphatische Gruppe ist, die durch O, S, CO, $SO_2$ oder $NR^5$ unterbrochen oder durch Halogen, Alkyl, Phenyl, Hydroxy, Alkoxy oder Aryloxy substituiert sein kann.

3. Verbindungen gemäss Anspruch 1 der Formel I, worin y=1 ist und Y eine Gruppe der Formel
$-(CH_2)_n-C(R^7)=CH_2$,
$-O-(CH_2)_n-C(R^7)=CH_2$,
$-O-CO-C(R^7)=CH_2$,
$-NH-CO-C(R^7)=CH_2$,
$-N(CH_2CH=CH_2)_2$,

$-CO-O-(CH_2)_n-C(R^7)=CH_2$,

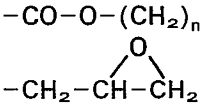 oder

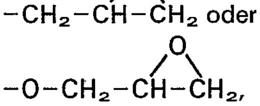

ist, worin n=0 oder 1 ist und $R^7$ Wasserstoff oder Methyl bedeutet.

4. Verbindungen gemäss Anspruch 1 der Formel I, worin y=2 ist und Y eine Epoxidgruppe oder eine Gruppe $-OH$, $-NHR^5$, $-COOH$, $-COOR^6$, $-COCl$, $-CH_2Hal$, $-SO_2Cl$ oder $-NCO$ ist.

5. Verbindungen gemäss Anspuch 1 der Formel I, worin y=1 ist und Y eine Epoxidgruppe oder eine Gruppe $-OH$, $-NHR^5$, $-COOH$, $-COOR^6$, $-COCl$, $-CH_2Hal$, $-SO_2Cl$ oder $-NCO$ ist.

6. Verbindungen gemäss Anspruch 1 der Formel I, worin x=1 ist,

$R^1$ $C_1$-$C_6$-Alkyl, $C_1$-$C_4$-Halogenalkyl, unsubstituiertes oder durch 1 oder 2 der Substituenten Cl, $C_1$-$C_4$-Alkyl, $C_1$-$C_4$-Alkoxy oder Nitro substituiertes Phenyl, 2-Furyl, 2-Thienyl, $C_1$-$C_4$-Alkoxy oder CN bedeutet,

$R^2$ eine der für $R^1$ gegebenen Bedeutungen hat oder Dialkylamino oder Morpholino ist, oder $R^1$ und $R^2$ zusammen mit den Atomen, an die sie gebunden sind, einen 5- oder 6-gliedrigen Ring bilden, der durch 1 oder 2 Benzoreste anneliert sein kann.

7. Verbindungen gemäss Anspruch 6 der Formel I, worin m=0 oder 1 ist, $R^1$ $C_1$-$C_4$-Alkyl, Trifluormethyl, Phenyl, Mono- oder Dichlorphenyl oder Methoxyphenyl ist, $R^2$ eine der für $R^1$ angegebenen Bedeutungen hat oder $-CN$ bedeutet, oder $R^1$ und $R^2$ zusammen mit dem C-Atom, an das sie gebunden sind, einen Inden-, Fluoren-, Tetralin- oder Dihydroanthracenring bilden.

8. Verbindungen gemäss Anspruch 1 der Formel I, worin x und y 1 sind und Z unsubstituiertes oder durch $C_1$-$C_{16}$-Alkyl oder Halogen substituiertes $C_6$-$C_{12}$-Arylen, $C_7$-$C_{12}$-Arylenalkylen, $C_8$-$C_{12}$-Arylendialkylen, Oxydiphenylen oder gerade- oder verzweigtkettiges $C_1$-$C_{12}$-Alkylen ist, das durch O, S, CO oder $NR^5$ unterbrochen oder durch Phenyl, Halogen, Phenoxy, $C_1$-$C_4$-Alkoxy oder OH substituiert sein kann.

9. Verbindungen gemäss Anspruch 8 der Formel I, worin Z Phenylen, Naphthylen, $C_2$-$C_6$-Alkylen, Benzylen oder eine Gruppe

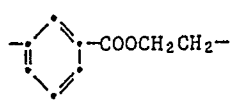 ist.

10. Polymere, die in Seitengruppen Reste der Formel IV

$$R^1-(\overset{\overset{O}{\|}}{C})_m-\underset{\underset{R^2}{|}}{C}=N-O-SO_2- \qquad (IV)$$

enthalten, worin m, $R^1$ und $R^2$ die in Anspruch 1 gegebenen Bedeutungen haben.

11. Polymere gemäss Anspruch 10, die durch Polymerisation einer Verbindung der Formel I des Anspruches 1 entstehen, worin Y eine ethylenisch ungesättigte Gruppe ist, oder durch Copolymerisation einer solchen Verbindung mit einer anderen ethylenisch ungesättigten Verbindung.

12. Polymere gemäss Anspruch 11, die durch Copolymerisation einer Verbindung der Formel I, worin Y eine ethylenisch ungesättigte Gruppe ist, mit einer oder mehreren α,β-ungesättigten Carbonsäuren oder deren Derivaten entstehen.

13. Copolymere gemäss Anspruch 12, worin die Comonomeren Acrylsäure, Methacrylsäure, oder ein Alkylester der Acryl- oder Methacrylsäure sind.

14. Polymere gemäss Anspruch 10, die durch Reaktion eines hydroxylgruppenhaltigen Polymeren mit einer Verbindung der Formel I des Anspruches 1, worin Y eine Gruppe $-COCl$, $-SO_2Cl$ oder $-NCO$ ist, entstehen.

15. Polymere gemäss Anspruch 14, die durch Reaktion eines Novolak-Harzes mit einer Verbindung der Formel I, worin Y $-COCl$ ist, entstehen.

16. Verwendung von Polymeren gemäss Anspruch 10 zur photochemischen Bilderzeugung.

17. Verfahren zur photochemischen Bilderzeugung durch Belichtung eines Filmes aus einem Polymeren gemäss Anspruch 10 mit kurzwelligem Licht unter Vorsatz einer Bildmaske und Entwicklung des Bildes mit einer wässerig-alkalischen Entwickler-Lösung.

18. Verfahren zur photochemischen Bilderzeugung durch Bestrahlung eines Filmes aus einem Polymeren gemäss Anspruch 10 mit Laserstrahlen und Entwicklung des Bildes mit einer wässerig-alkalischen Entwickler-Lösung.

19. Verfahren zum Härten von säurehärtbaren Einbrennlacken durch Zusatz mindestens einer Verbindung der Formel I des Anspruches 1 oder eines daraus hergestellten Polymeren und Bestrahlung des Lackfilmes mit kurzwelligem Licht und anschliessendem Erwärmen.

20. Verfahren gemäss Anspruch 19 durch Zusatz eines Polymeren gemäss Anspruch 10, Bestrahlung des Lackfilmes mit UV-Licht und Erwärmen auf 80-120° C.

## Claims

1. A compound of formula I

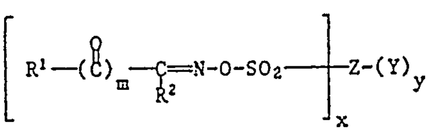 (I)

wherein m is 0 or 1, x is 1 or 2 and y is 1 or 2,

$R^1$ is $C_1$-$C_{12}$alkyl, $C_1$-$C_4$haloalkyl, $C_5$-$C_{12}$cycloalkyl, $C_6$-$C_{10}$aryl which is unsubstituted or substituted by one or more of the substituents halogen, $C_1$-$C_{16}$alkyl, $C_1$-$C_4$alkoxy, phenoxy, phenyl and nitro, or $R^1$ is furyl, thienyl, $C_7$-$C_{12}$aralkyl, $C_1$-$C_8$alkoxy, $C_5$-$C_8$cycloalkoxy, phenoxy or CN,

$R^2$ has one of the meanings indicated for $R^1$, or is $C_2$-$C_6$alkanoyl, benzoyl, $C_2$-$C_5$alkoxycarbonyl, phenoxycarbonyl, $-N(R^3)(R^4)$, morpholino or piperidino, or $R^1$ and $R^2$, together with the atoms to which they are attached, form a 5- to 8-membered ring which may be fused with 1 or 2 benzo radicals, $R^3$ being hydrogen, $C_1$-$C_{12}$alkyl, phenyl, $C_2$-$C_6$alkanoyl or benzoyl and $R^4$ being hydrogen, $C_1$-$C_{12}$alkyl or cyclohexyl,

Y is a polymerisable ethylenically unsaturated group or epoxide group or an $-OH$, $-NHR^5$, $-COOH$, $-COOR^6$, $-COCl$, $-CH_2Hal$, $-SO_2Cl$ or $-NCO$ group, in which $R^5$ is hydrogen, $C_1$-$C_6$alkyl or phenyl, $R^6$ is $C_1$-$C_6$alkyl or phenyl and Hal is chlorine or bromine, and

Z is an (x+y)-valent organic connecting member which links the sulfo radical $-SO_2-$ with the group Y.

2. A compound according to claim 1 of formula 1, wherein Z is an (x+y)-valent aromatic, aliphatic, cycloaliphatic or araliphatic group which may be interrupted by O, S, CO, $SO_2$ or $NR^5$ or may be substituted by halogen, alkyl, phenyl, hydroxy, alkoxy or aryloxy.

3. A compound according to claim 1 of formula 1, wherein y is 1 and Y is a group of the formula

$-(CH_2)_n-C(R^7)=CH_2$,
$-O-(CH_2)_n-C(R^7)=CH_2$,
$-O-CO-C(R^7)=CH_2$,
$-NH-CO-C(R^7)=CH_2$,
$-N(CH_2CH=CH_2)_2$,
$-CO-O-(CH_2)_n-C(R^7)=CH_2$,

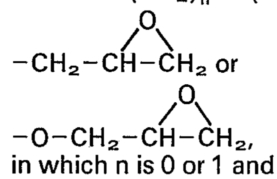

in which n is 0 or 1 and $R^7$ is hydrogen or methyl.

4. A compound according to claim 1 of formula I, wherein y is 2 and Y is an epoxide group or an $-OH$, $-NHR^5$, $-COOH$, $-COOR^6$, $-COCl$, $-CH_2Hal$, $-SO_2Cl$ or $-NCO$ group.

5. A compound according to claim 1 of formula I, wherein y is 1 and Y is an epoxide group or an $-OH$, $-NHR^5$, $-COOH$, $-COOR^6$, $-COCl$, $-CH_2Hal$, $-SO_2Cl$ or $-NCO$ group.

6. A compound according to claim 1 of formula I, wherein x is 1, $R^1$ is $C_1$-$C_6$alkyl, $C_1$-$C_4$haloalkyl, phenyl which is unsubstituted or substituted by 1 or 2 of the substituents chlorine, $C_1$-$C_4$alkyl, $C_1$-$C_4$alkoxy and nitro, or is 2-furyl, 2-thienyl, $C_1$-$C_4$alkoxy or $-CN$, $R^2$ has one of the meanings indicated for $R^1$ or is dialkylamino or morpholino, or $R^1$ and $R^2$, together with the atoms to which they are attached, form a 5- or 6-membered ring which may be fused with 1 or 2 benzo radicals.

7. A compound according to claim 6 of formula I, wherein m is 0 or 1, $R^1$ is $C_1$-$C_4$alkyl, trifluoromethyl, phenyl, monochlorophenyl, dichlorophenyl or methoxyphenyl, $R^2$ has one of the meanings indicated for $R^1$ or is $-CN$, or $R^1$ and $R^2$, together with the carbon atom to which they are attached, form an indene, fluorene, tetraline or dihydroanthracene ring.

8. A compound according to claim 1 of formula I, wherein x and y are 1 and Z is $C_6$-$C_{12}$arylene which is unsubstituted or substituted by $C_1$-$C_{16}$alkyl or halogen, or is $C_7$-$C_{12}$arylenealkylene, $C_8$-$C_{12}$arylenedialkylene, oxydiphenylene, or straight-chain or branched $C_1$-$C_{12}$alkylene which may be interrupted by O, S, CO or $NR^5$ or may be substituted by phenyl, halogen, phenoxy, $C_1$-$C_4$alkoxy or OH.

9. A compound according to claim 8 of formula 1, wherein Z is phenylene, naphthylene, $C_2$-$C_6$alkylene, benzylene or a

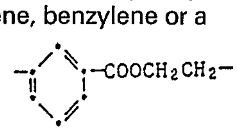 group.

10. A polymer containing in side groups radicals of formula IV

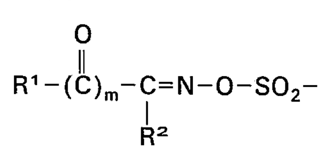 (IV)

wherein m, $R^1$ and $R^2$ are as defined in claim 1.

11. A polymer according to claim 10, which is formed by polymerisation of a compound of formula I according to claim 1, in which Y is an ethylenically unsaturated group, or by copolymerisation of such a compound with another ethylenically unsaturated compound.

12. A polymer according to claim 11, which is formed by copolymerisation of a compound of formula I, wherein Y is an ethylenically unsaturated group, with one or more α,β-unsaturated carboxylic acids or with derivatives thereof.

13. A copolymer according to claim 12, wherein the comonomers are acrylic acid, methacrylic acid, or an alkyl ester of acrylic acid or methacrylic acid.

14. A polymer according to claim 10, which is formed by reacting a hydroxyl group-containing polymer with a compound of formula I according to claim 1, wherein Y is a $-COCl$, $-SO_2Cl$ or $-NCO$ group.

15. A polymer according to claim 14, which is formed by reacting a novolak resin with a compound of formula I, wherein Y is $-COCl$.

16. Use of a polymer according to claim 10 for the photochemical production of images.

17. A process for the photochemical production of an image, which process comprises exposing a film made of a polymer according to claim 10 to shortwave light through a photomask and developing the image with an aqueous alkaline developer solution.

18. A process for the photochemical production of an image, which process comprises irradiating a film made of a polymer according to claim 10 with laser beams and developing the image with an aqueous alkaline developer solution.

19. A process for the curing of an acid-curable stoving enamel by adding at least one compound of formula I of claim 1 or a polymer prepared therefrom and irradiating the coating film with shortwave light and subsequently heating said film.

20. A process according to claim 19, which comprises adding a polymer according to claim 10, irradiating the coating film with UV light and heating said film to 80 to 120° C.

## Revendications

1. Composés qui répondent à la formule I:

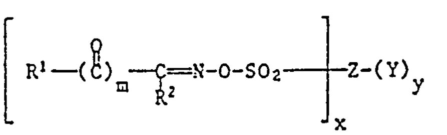

(I)

dans laquelle:

m est égal à 0 ou à 1, x à 1 ou à 2 et y à 1 ou à 2,

$R^1$ représente un alkyle en $C_1$-$C_{12}$, un halogénoalkyle en $C_1$-$C_4$, un cycloalkyle en $C_5$-$C_{12}$, un aryle en $C_6$-$C_{10}$ non substitué ou porteur d'un ou plusieurs substituants pris dans l'ensemble constitué par les halogènes, les alkyles en $C_1$-$C_{16}$, les alcoxy en $C_1$-$C_4$, le phénoxy, le phényle et le nitro, un furyle, un thiényle, un aralkyle en $C_7$-$C_{12}$, un alcoxy en $C_1$-$C_8$, un cycloalcoxy en $C_5$-$C_8$, un phénoxy ou un CN,

$R^2$ a l'une des significations qui viennent d'être données pour $R^1$ ou représente un alcanoyle en $C_2$-$C_6$, un benzoyle, un alcoxycarbonyle en $C_2$-$C_5$, un phénoxycarbonyle, un radical $-N(R^3)(R^4)$, un morpholino ou un pipéridino, ou encore $R^1$ et $R^2$ forment ensemble, et avec les atomes auxquels ils sont liés, un cycle qui comporte de 5 à 8 maillons et qui peut être condensé à 1 ou 2 radicaux benzo, le symbole $R^3$ désignant l'hydrogène, un alkyle en $C_1$-$C_{12}$, un phényle, un alcanoyle en $C_2$-$C_6$ ou un benzoyle et le symbole $R^4$ l'hydrogène, un alkyle en $C_1$-$C_{12}$ ou un cyclohexyle,

Y représente un radical éthylénique polymérisable, un radical époxydique ou un radical $-OH$, $-NHR^5$, $-COOH$, $-COOR^6$, $-COCl$, $-CH_2Hal$, $-SO_2Cl$ ou $-NCO$, le symbole $R^5$ désignant l'hydrogène, un alkyle en $C_1$-$C_6$ ou un phényle, le symbole $R^6$ un alkyle en $C_1$-$C_6$ ou un phényle en Hal le chlore ou le brome, et

Z représente un groupement organique de liaison, de valence égale à (x+y), qui unit le radical sulfonyle $-SO_2-$ au radical Y.

2. Composés de formule I selon la revendication 1 dans lesquels Z représente un radical aromatique, aliphatique, cycloaliphatique ou araliphatique, de valence égale à (x+y), qui peut être interrompu par O, S, CO, $SO_2$ ou $NR^5$ ou qui peut porter comme substituant un halogène, un alkyle, un phényle, un hydroxy, un alcoxy ou un aryloxy.

3. Composés de formule I selon la revendication 1 dans lesquels y est égal à 1 et Y représente un radical répondant à l'une des formules suivantes:

$-(CH_2)_n-C(R^7)=CH_2$,

$-O-(CH_2)_n-C(R^7)=CH_2$,

$-O-CO-C(R^7)=CH_2$,

$-NH-CO-C(R^7)=CH_2$,

$-N(CH_2CH=CH_2)_2$,

$-CO-O-(CH_2)_n-C(R^7)=CH_2$,

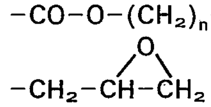 et

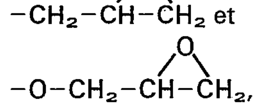,

où n est égal à 0 ou à 1 et $R^7$ représente l'hydrogène ou un méthyle.

4. Composés de formule I selon la revendication 1 dans lesquels y est égal à 2 et Y représente un radical époxydique ou un radical $-OH$, $-NHR^5$, $-COOH$, $-COOR^6$, $-COCl$, $-CH_2Hal$, $-SO_2Cl$ ou $-NCO$.

5. Composés de formule I selon la revendication 1 dans lesquels y est égal à 1 et Y représente un radical époxydique ou un radical $-OH$, $-NHR^5$, $-COOH$, $-COOR^6$, $-COCl$, $-CH_2Hal$, $-SO_2Cl$ ou $-NCO$.

6. Composés de formule I selon la revendication 1 dans lesquels x est égal à 1, $R^1$ représente un alkyle en $C_1$-$C_6$, un halogéno-alkyle en $C_1$-$C_4$, un phényle non substitué ou porteur d'un ou deux

substituants pris dans l'ensemble constitué par Cl, les alkyles en $C_1$-$C_4$, les alcoxy en $C_1$-$C_4$ et le nitro, un furyle-2, un thiényle-2, un alcoxy en $C_1$-$C_4$ ou un CN, $R^2$ a l'une des significations qui viennent d'être données pour $R^1$ ou représente un radical dialkylamino ou morpholino, ou encore $R^1$ et $R^2$ forment ensemble, et avec les atomes auxquels ils sont liés, un cycle à 5 ou 6 maillons, qui peut être condensé à 1 ou 2 noyaux benzo.

7. Composés de formule I selon la revendication 6 dans lesquels m est égal à 0 ou à 1, $R^1$ représente un alkyle en $C_1$-$C_4$, un trifluorométhyle, un phényle, un monochlorophényle, un dichlorophényle ou un méthoxyphényle, $R^2$ a l'une des significations qui ont été données pour $R^1$ ou représente un radical cyano, ou encore $R^1$ et $R^2$ forment ensemble, et avec l'atome de carbone auquel ils sont liés, un cycle d'indène, de fluorène, de tétraline ou de dihydro-anthracène.

8. Composés de formule I selon la revendication 1 dans lesquels x et y sont égaux chacun à 1, et Z représente un radical arylène en $C_6$-$C_{12}$ non substitué ou porteur d'un alkyle en $C_1$-$C_{16}$ ou d'un halogène, un arylène-alkylène en $C_7$-$C_{12}$, un arylène-dialkylène en $C_8$-$C_{12}$, un oxy-diphénylène ou un alkylène en $C_1$-$C_{12}$, linéaire ou ramifié, qui peut être interrompu par O, S, CO ou $NR^5$ ou qui peut porter comme substituant un phényle, un halogène, un phénoxy, un alcoxy en $C_1$-$C_4$ ou un $-OH$.

9. Composés de formule I selon la revendication 8 dans lesquels Z représente un phénylène, un naphtylène, un alkylène en $C_2$-$C_6$, un benzylène ou un radical:

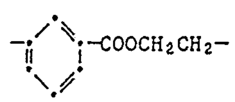

10. Polymères qui contiennent, dans des groupes latéraux, des radicaux répondant à la formule IV:

$$R^1-\overset{\overset{O}{\|}}{(C)}_m-\underset{\underset{R^2}{|}}{C}=N-O-SO_2- \qquad (IV)$$

dans laquelle m, $R^1$ et $R^2$ ont les significations données à la revendication 1.

11. Polymères selon la revendication 10 qui se forment par polymérisation d'un composé de formule I selon la revendication 1 dans lequel Y représente un radical éthylénique, ou par copolymérisation d'un tel composé avec un autre composé éthylénique.

12. Polymères selon la revendication 11 qui se forment par copolymérisation d'un composé de formule I dans lequel Y représente un radical éthylénique, avec un ou plusieurs acides carboxyliques insaturés en α,β ou leurs dérivés.

13. Copolymères selon la revendication 12 dans lesquels les comonomères sont pris dans l'ensemble constitué par l'acide acrylique, l'acide méthacrylique et les esters alkyliques de ces acides.

14. Polymères selon la revendication 10 qui se forment par réaction d'un polymère contenant un radical hydroxy avec un composé de formule I selon la revendication 1 dans lequel Y représente un radical $-COCl$, $-SO_2Cl$ ou $-NCO$.

15. Polymères selon la revendication 14 qui se forment par réaction d'un résine novolaque avec un composé de formule I dans lequel Y représente un radical $-COCl$.

16. Application de polymères selon la revendication 10 pour la production photochimique d'images.

17. Procédé pour produire des images photochimiquement, procédé selon lequel on expose une pellicule en un polymère selon la revendication 10, derrière un masque portant une image, à un rayonnement de courte longueur d'onde et on développe l'image au moyen d'une solution aqueuse alcaline d'un révélateur.

18. Procédé pour la production photochimique d'images, selon lequel on expose à des faisceaux lasers une pellicule en un polymère selon la revendication 10 et on développe l'image au moyen d'une solution aqueuse alcaline d'un révélateur.

19. Procédé pour durcir des peintures et vernis à cuire durcissables par des acides, selon lequel on ajoute au moins un composé de formule I selon la revendication 1 ou un polymère préparé à partir d'un tel composé, on expose la pellicule de peinture ou de vernis à un rayonnement de courte longueur d'onde et ensuite on la chauffe.

20. Procédé selon la revendication 19, selon lequel on ajoute un polymère selon la revendication 10, on expose la pellicule de peinture ou de vernis à un rayonnement ultra-violet et on la chauffe à une température de 80 à 120° C.